(19) Europäisches Patentamt

European Patent Office

Office européen des brevets

(11) EP 1 629 818 A1

(12) EUROPEAN PATENT APPLICATION

(43) Date of publication:
01.03.2006 Bulletin 2006/09

(51) Int Cl.:
A61H 1/00 (2006.01)          A61H 37/00 (2006.01)
A61B 5/053 (2006.01)

(21) Application number: 05018709.5

(22) Date of filing: 29.08.2005

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 30.08.2004 JP 2004250657

(71) Applicant: Omron Healthcare Co., Ltd.
Kyoto 615-0084 (JP)

(72) Inventor: Kasai, Eiji
c/o KETEC CO., Ltd.
Kyoto 617-0002 (JP)

(74) Representative: Kilian, Helmut
Wilhelms, Kilian & Partner
Patentanwälte
Eduard-Schmid-Strasse 2
81541 München (DE)

(54) **Massager**

(57) A massager comprising a back rest portion, massage heads (402a-402d) provided in the back rest portion for massaging the user, a treatment section (40) for moving a pair of massage head arms (403R,403L) for supporting the massage heads according to the massaging operation, a sensor section (100) for sensing bio-signals of the user depending on impedance changes between the pair of massage head arms, and a control section (13) for controlling the massaging operation of the massage heads, wherein the control section moves the treatment section and judges the position of a discomfort site of the user by detecting an extreme value of the bio-signal in a predetermined range.

FIG. 1

**Description**

Background of the invention

Field of the invention

The present invention relates to a massager. More particularly, the invention relates to a massager using a sensor for sensing a position of a discomfort site of the user.

Description of the related art

Conventionally, there are known massagers capable of massaging without giving discomfort or pain to the user, and some of them are provided with sensors for sensing discomfort site (pain spot) for the user.
The sensor for sensing discomfort site (pain spot) for the user includes the following examples.
Japanese Patent Application Laid-Open (JP-A) No. 2001-269380 discloses a massager for controlling massaging operations by sensing stiffness of treated area contacting with a treatment applicator on the basis of pressure changes detected by pressure detecting means.
Japanese Patent Application Laid-Open (JP-A) No. 5-31147 discloses a massager comprising pressure detecting means for detecting the pressure of treatment applicator on massage site, and means for detecting the angle of treatment applicator to massage site, thereby preventing uncomfortable massaging operation due to excessive projection of treatment applicator.
Japanese Patent No. 3128260 discloses a massager comprising pressure detecting means for detecting the pressure of roller on massage site, thereby detecting the positions of neck, shoulder, back and waist of the user from the obtained pressure curve, and massaging on the basis of these positions.
In the conventional massagers, however, although positions of characteristic profiles of neck, shoulder, shoulder blade, back, sidebone and waist canbe detected, sensible sites (pain spots) felt by the user by injury or wound cannot be detected.
In particular, in the case of a massager designed to detect a specific site from the pressure applied to a treatment applicator, when the treatment applicator touches the pain spot, the user tries to remove the body from the treatment applicator, and the pressure applied to the pain spot is decreased temporarily. Accordingly, the pressure detecting means determines that the pressure is insufficient on the pain spot, and a higher pressure is applied to the pain spot, and hence it is more uncomfortable for the user.
The massager disclosed in Japanese Patent Application Laid-Open (JP-A) No. 2001-269380 requires a plurality of pressure sensors, and the structure is complicated and the cost is increased. Also fluctuations of sensing increase depending on the sitting manner of the user.
The massager disclosed in Japanese Patent Application Laid-Open (JP-A) No. 5-31147 or Japanese Patent No. 3128260 also requires a plurality of sensors such as strain gauge, load sensor or angle sensor, and the structure of the massage unit is complicated, and the cost of sensing is increased.

Summary of the Invention

The invention is devised in the light of the prior art, and it is hence an object thereof to provide a massager simple in structure and high in precision, and capable of sensing a site felt discomfort for the user (discomfort site).
To achieve the above-mentioned obj ect, the present invention provides a massager comprising:

a back rest portion for supporting the back of the user,
treatment applicators provided in the back rest portion for massaging the user,
a treatment section for moving a pair of electrodes supporting the treatment applicators according to the massaging operation,
a bio-signal sensor for sensing bio-signals of the user depending on impedance changes between the pair of electrodes, and
a control section for controlling the massaging operation of the treatment applicators on the basis of the bio-signals, wherein the bio-signal sensor has:

an oscillation section for supplying a high frequency signal,
the pair of electrodes disposed to be approached or touched by the user for receiving the high frequency signal from the oscillation section,
a sensor section for sensing changes of the impedance occurring in the electrodes receiving the high frequency signal when the user approaches or touches the electrodes, and
a signal processor section for processing from a signal corresponding to the sensed impedance changes to a signal depending on a desired bio-signal, and
the control section moves the treatment section and detect an extreme value of signal corresponding to the bio-signal in a predetermined range, and judges the position of discomfort site of the user.

Preferably, there is provided a massager, wherein the extreme value is a minimum value sensed after a first peak.

Herein, the discomfort site is anywhere in the body felt discomfort for the user at the time of massaging, for example, wound or bruise position, protruding positions with less muscle or fat such as shoulder blade, and other positions felt painful (pain spot) individually in ordinary massaging operation.

According to these configurations, positions of discomfort sites are judged automatically without requiring setting of positions of discomfort sites by the user, and comfortable massaging operation is realized without loading the user with any extra burden or discomfort.

Preferably, there is provided A massager, wherein the control section controls so as not to massage the position of discomfort site when the position of discomfort site is judged.

Preferably, there is provided a massager, wherein the control section controls to weaken the pressure of treatment applicators in massaging operation at the position of discomfort site when the position of discomfort site is judged.

According to these configurations, massaging operations can be executed by avoiding unpleasant positions for the user or weakening the pressure of the treatment applicator on such positions, so that the user can enjoy comfortable massaging.

Preferably, there is provided a massager, wherein the control section can judge a plurality of the positions of discomfort site.

According to this configuration, if the user has a plurality of discomfort sites, comfortable massaging operation is realized.

Preferably, there is provided a massager, wherein the control section does not judge the position of discomfort site of the user when the extreme value is not lower than a predetermined reference value.

According to this configuration, if a signal corresponding to a bio-signal obtained from a bio-signal sensor is in a measuring range in which change of output value from bio-signal sensor is - due to an individual body type of the user or the like, it prevents misjudging of position of discomfort site (pain spot) so that the massager does not operate according to a wrong position of discomfort site (pain spot).

Preferably, there is provided a massager, wherein the control section judges the position of discomfort site again when the inclination of the back rest portion is changed, and controls the massaging operation by the treatment applicator on the basis of the newly judged position of discomfort site.

According to this configuration, even if the inclination of back rest portion is changed (reclined) , comfortable massaging operation is realized by sensing the position of discomfort site again.

Preferably, there is provided a massager, wherein the control section corrects the position of discomfort site, when the inclination of the back rest portion is changed, according to the changed inclination, and controls the massaging operation by the treatment applicator on the basis of the corrected position of discomfort site.

According to this configuration, to sense the position of discomfort site again, it is not required to move the treatment section to initial position, so that the massage treating time can be shortened.

Preferably, there is provided a massager, wherein the control section judges the position of discomfort site again in a predetermined time after judging the position of discomfort site, and controls the massaging operation by the treatment applicator on the basis of the newly judged position of discomfort site.

According to this configuration, the position of discomfort site is always judged accurately, and optimum massaging operation is realized.

Preferably, there is provided a massager, wherein the electrodes are arms rotatable with respect to the treatment section for supporting the plurality of treatment applicators, and
the plurality of treatment applicators are disposed in a line nearly parallel to the moving direction of the treatment section on both sides of the center of rotation of the arms.

According to this configuration, among the plurality of treatment applicators supported by the arm, if one treatment applicator provided at the moving direction side of the treatment section touches the user, the reactive force from the user acts on the abutting treatment applicator so that the arm is rotated, and it comes closer to the user together with other treatment applicator provided at the opposite side of the abutting treatment applicator, so that the bio-signal from the user can be sensed precisely, and the position of discomfort site of the user can be judged more accurately.

Preferably, there is provided a massager, wherein the sensor section generates and supplies a signal expressing changes of reflected wave level of the high frequency signal corresponding to the impedance changes occurring between the electrodes depending on the body type of the user.

Preferably, there is provided a massager, wherein the sensor section outputs a reflected wave signal depending on impedance changes of real number and imaginary number occurring between the electrodes depending on the body type of the user.

Preferably, there is provided a massager, wherein the frequency of the high frequency signal is in a range of 10 MHz to 300 MHz.

According to the invention, a massager simple in structure and high in precision, and capable of sensing a site felt discomfort for the user can be realized.

Brief description of the drawings

Fig. 1 is a schematic block diagram of a sensor section included in an electrical configuration of a massager according to a present embodiment;

Fig. 2 is a general front view of outline configuration of the massager according to the present embodiment;

Fig. 3 is a general side view of outline configuration of the massager according to the present embodiment;

Fig. 4 is a front view of an elevating treatment unit according to the present embodiment;

Fig. 5 is a rear view of the elevating treatment unit according to the present embodiment;

Fig. 6 is a perspective view of a treatment section of the massager according to the present embodiment;

Fig. 7 is a schematic block diagram of electrical configuration of the massager according to the present embodiment;

Fig. 8 is a graph showing an example of output fluctuations from the sensor section obtained in the massager according to the present embodiment;

Fig. 9 is a flowchart of operation of the massager according to example 1;

Fig. 10 is a flowchart of operation of the massager according to example 2; and

Fig. 11 is a flowchart of operation of the massager according to example 3.

Detailed description of the preferred embodiments

Referring now to the drawings, the best mode for carrying out the invention is specifically described below. In the illustrated embodiments, however, dimensions, material, shape, function, and relative positions of the constituent parts are not particularly specified in the scope of the invention unless otherwise noted. Similarly, quality, shape and functions of the materials once explained are the same in the following embodiments unless otherwise specified particularly.

(Overall configuration of massager)

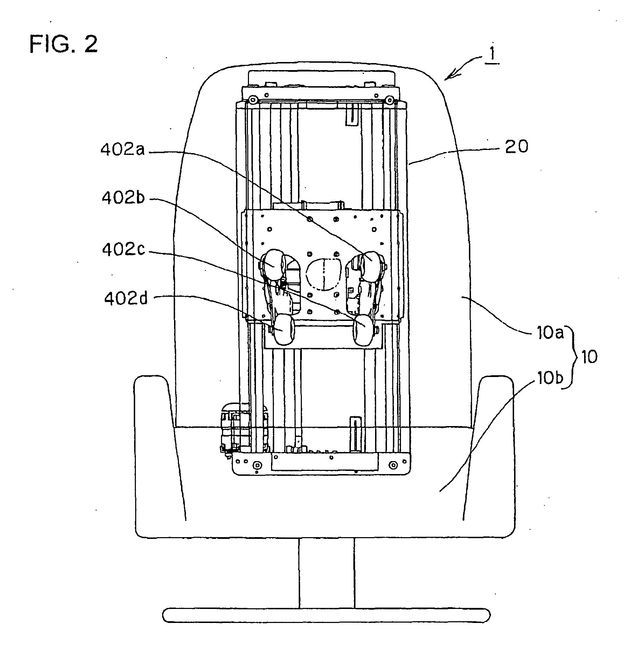

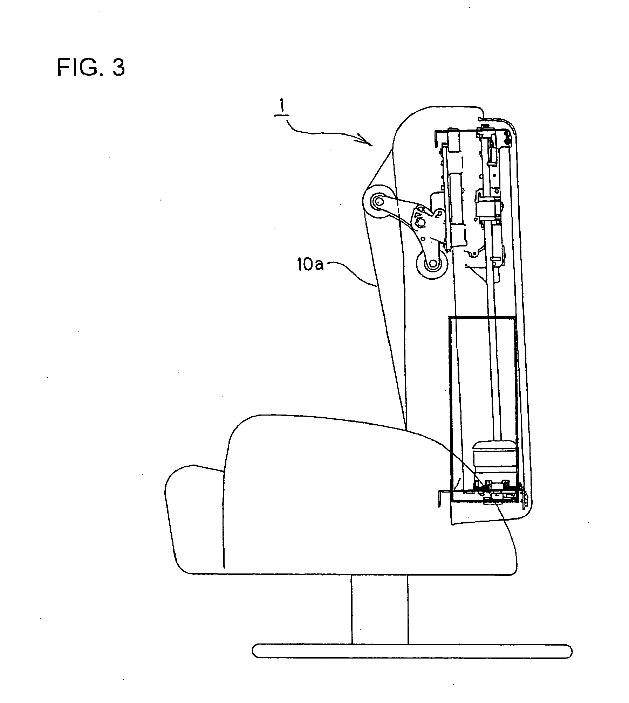

Fig. 2 is a general front view of outline configuration of massager for carrying out the invention favorably. Fig. 3 is a general side view of outline configuration of massager for carrying out the invention favorably.

A massager 1 comprises a reclinable chair 10, and an elevating treatment unit 20. The chair 10 consists of a back rest portion 10a for supporting the back of the user, and a seat 10b, and the elevating treatment unit 20 is assembled in the back rest portion 10a.

The elevating treatment unit 20 has massage heads (treatment applicators) 402a to 402d protruding toward the surface side covered with cover sheet of the back rest portion 10a, and the parts of body are massaged by the massage heads 402a to 402d.

Massage heads are provided in the back rest portion 10a and massage the user. More specifically, a pair of right and left massage heads 402a, 402b (first treatment applicators) are provided above along the spine direction, and a pair of right and left massage heads 402c, 402d (second treatment applicators) are provided beneath.

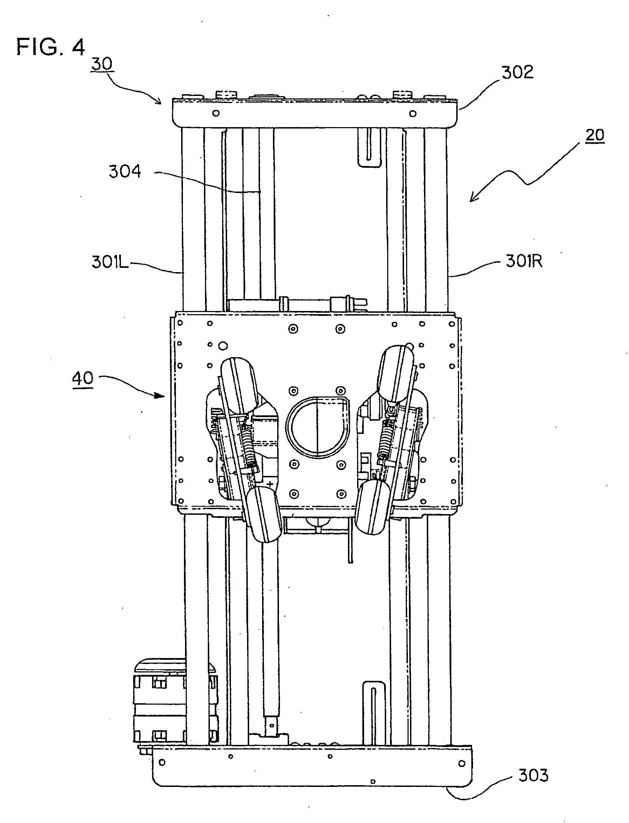

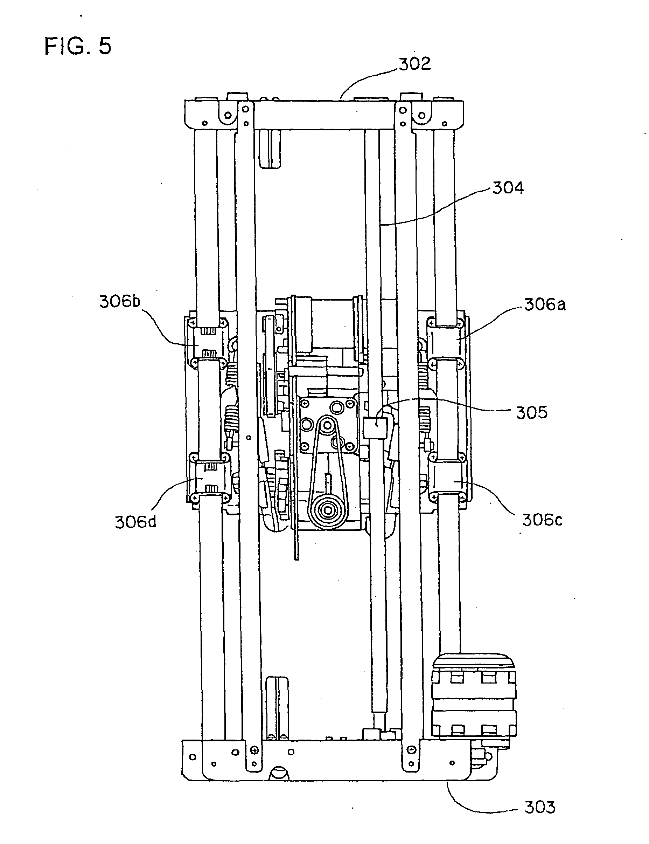

Fig. 4 is a front view of the elevating treatment unit. Fig. 5 is a rear view of the elevating treatment unit.

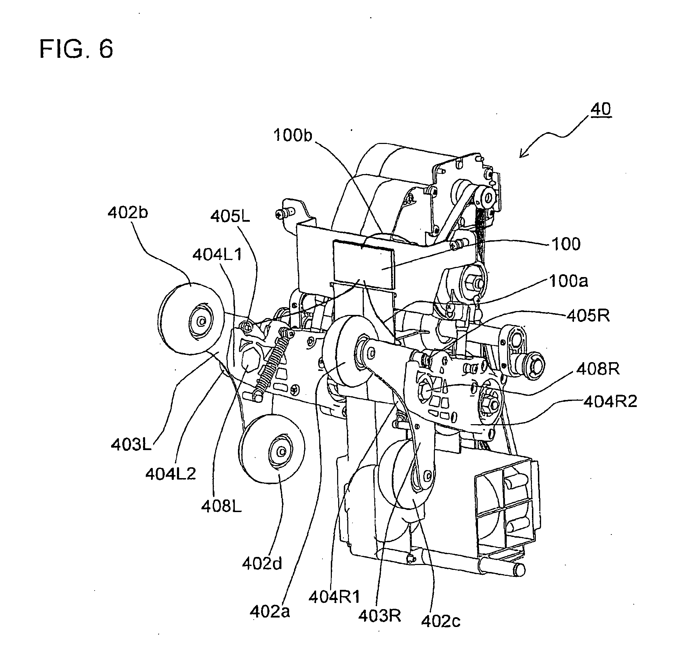

The elevating treatment unit 20 has an elevation section 30 and a treatment section 40, and moves arms 403R, 403L (refer to Fig. 6) functioning as a pair of electrodes supporting the treatment applicators along with the massaging operation. The elevation section 30 is, as shown in Fig. 4, composed of a pair of guide pipes 301R, 301L of circular section disposed along the back rest portion 10a, a elevation-dedicated threaded shaft 304 disposed parallel to the guide pipes between the both guide pipes, and a pair of upper and lower guide pipe holders 302, 303 orthogonal to the guide pipes 301R, 301L.

The guide pipes 301R, 301L are fixed on the guide pipe holders 302, 303, and the elevation-dedicated threaded shaft 304 is supported rotatably.

The treatment section 40 is, as shown in Fig. 5, supported by elevating guides 306a, 306b, 306c, 306d for movably holding in axial direction of guide pipes 301R, 301L, and an elevating nut holder (not shown) for holding an elevating nut 305 engaged with the outer periphery of elevation-dedicated threaded shaft 304.

In the treatment section 40, as the elevating nut 305 is driven up and down by rotation of the elevation-dedicated threaded shaft 304, the elevating nut holder and the treatment section 40 for supporting it are moved up and down along the guide pipes 301R, 301L.

(Outline configuration of treatment section)

An outline configuration of the treatment section 40 is explained. Fig. 6 is a perspective view of treatment section of massager capable of carrying out the invention favorably.

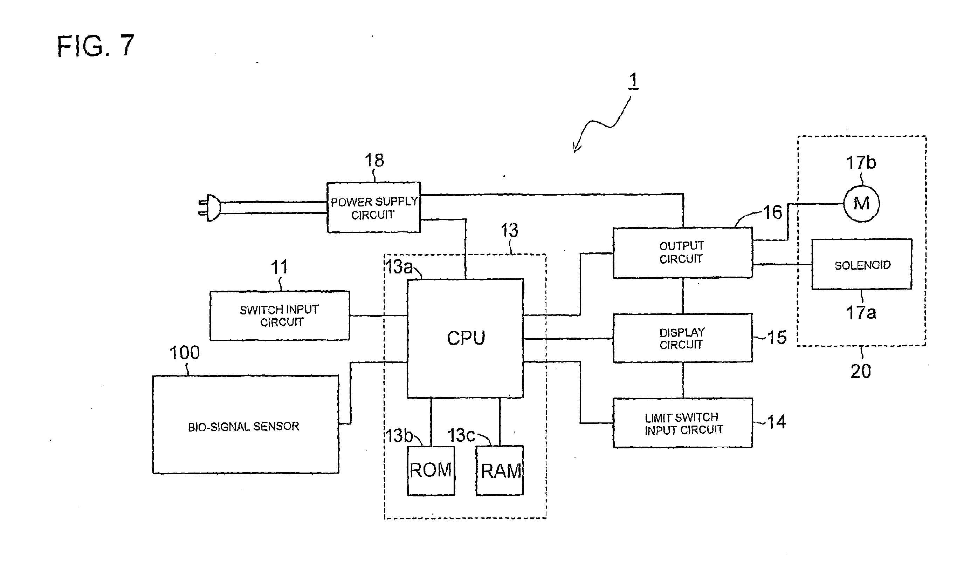

The treatment section 40 has four massage heads 402a to 402d, substantially V-shaped massage head arms 403R, 403L, a bio-signal sensor (referred to as sensor section, hereinafter) 100 for oscillating a signal of predetermined frequency, and sensing a bio-signal of the user from impedance change betweenmassage head arms 403R and 403L, and a control section 13 (refer to Fig. 7) for controlling the massaging operation of the massager 1 including the treatment section 40 on the basis of the bio-signal output from the sensor section 100.

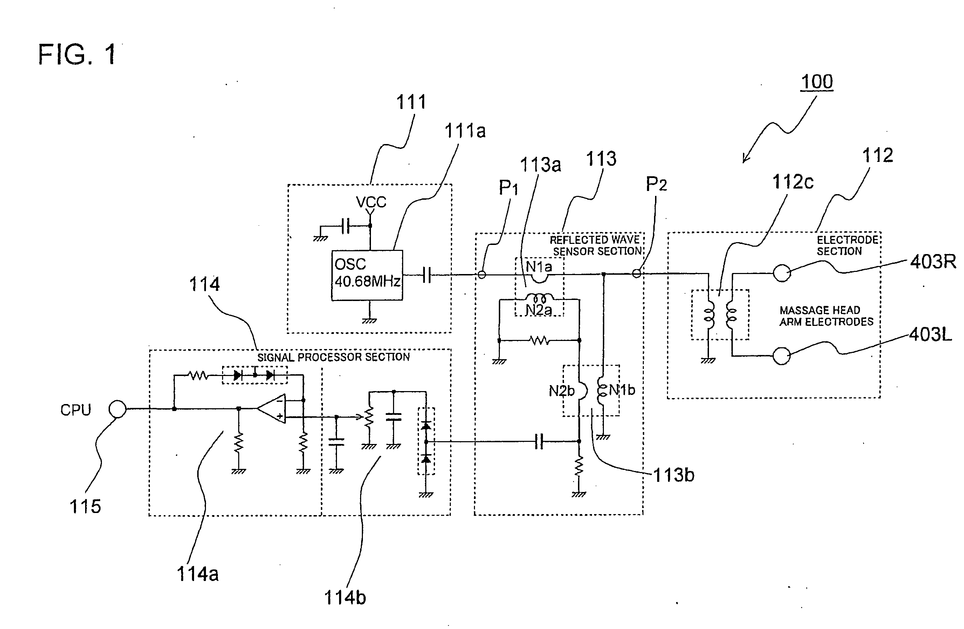

The massage head arms 403R, 403L function as electrodes for sensing the shape and presence or absence of parts of body, and are coupled to a reflected wave sensor section 113 (refer to Fig. 1) of sensor section 100 by way of a pair of feeding lines 100a provided for supplying high frequency signal from an oscillation section 111 (refer to Fig. 1). One end of the feeding line 100a is coupled to the massage head arms 403R, 403L by screw or solder.

The massage head arms 403R, 403L pivotally support the massage heads 402a to 402d, two by each, rotatably at the leading ends.

The base end of massage head arm 403R is sandwiched by members 404R1, 404R2 of arm support member 404R and supported by a pivotal shaft 408R. On the other hand, the base end of massage head arm 403L is sandwiched by members 404L1, 404L2 of arm support member 404L and supported by a pivotal shaft 408L. The massage head arms 403R, 403L are thereby rotatable on the treatment section 40.

The plurality of massage heads provided on the massage head arms 403R, 403L are provided nearlyparallel to the moving direction of the treatment section 40.

The massage head arms 403R, 403L are provided with stoppers 405R, 405L for stopping rotation respectively.

The massage head arms 403R, 403L are rotatable on the treatment section 40, and are arms for supporting a plurality of massage heads, and the plurality of mas sage heads are preferably arranged nearly parallel to the moving direction of the treatment section 40 about the center of rotation of massage head arms 403R, 403L.

In this configuration, among the plurality of massage heads 402a to 402d supported by the massage head arms 403R, 403L, when the massage heads 402c, 402d provided at the moving direction side of the treatment section 40 abut against the user, the massage head arms 403R, 403L supporting the abutting massage heads 402c, 402d and other massage heads 402a, 402b arranged in a direction toward the opposite side of the moving direction of the treatment section 40 from the massage heads 402c, 402d come closer to the user, so that the bio-signal from the user can be sensed precisely.

(Control circuit of massager)

Fig. 7 is a schematic block diagram of electrical configuration of massager according to the invention.

As shown in Fig. 7, the massager 1 mainly includes a switch input circuit 11, a sensor section (bio-signal sensor) 100, a control section 13 for controlling the operation of the massager 1, a limit switch input circuit 14, a display circuit 15, an output circuit 16, an elevating treatment unit 20, and a power supply circuit 18.

The control section 13 includes a central processing unit (CPU) 13a, a read only memory (ROM) 13b, and a random access memory (RAM) 13c. The elevating treatment unit 20 is assembled in the back rest portion 10a, and includes a solenoid 17a and a motor 17b.

The switch input circuit 11 includes, although not shown, power switch, and other key switches for specifying motions of the elevating treatment unit 20, such as rubbing, tapping, ascending, and descending motions.

The sensor section 100 is a bio-signal sensor for sensing bio-signals from the user, and bio-signals of pulse rate and respiration rate are produced and given to the CPU 13a of the control section 13. Bio-signals are different depending on the body type, muscles and fats and the like of the user, and by processing the bio-signals, the body type, body fat percentage and others of the users are indirectly measured and calculated.

When using the massager 1, the user turns on the power switch, not shown, of the switch input circuit 11, and the CPU 13a responds to it and starts the power supply circuit 18, and the power supply circuit 18 supplies power source to the parts of the massager 1.

The CPU 13a, depending on the instruction from the user through the switch input circuit 11, generates a signal for driving the elevating treatment unit 20, and applies to the output circuit 16. Depending on this signal, the output circuit 16 drives the solenoid 17a and motor 17b for composing the elevating treatment unit 20, and realizes the operation instructed by the user.

The CPU 13a also drives the display circuit 15, and displays the instruction by the user and other necessary information to the user.

The limit switch input circuit 14 is a circuit for specifying the range of vertical motion of the treatment section 40 in the elevating treatment unit 20 on the back rest portion 10a, and when the treatment section 40 ascends to reach the upper limit position, or descends to reach the lower limit position, the limit switch is actuated, and the vertical motion of the treatment section 40 is stopped.

The entire motion of the massager in Fig. 2 on the basis of the bio-signal sensed by the treatment section 40 of the invention is described later.

(Circuit configuration of the sensor section)

Fig. 1 is a schematic block diagram of the sensor section included in electrical configuration of the massager shown in Fig. 7.

As shown in Fig. 1, the sensor section 100 comprises an oscillation section 11, an electrode section 112, a reflected wave sensor section 113, a signal processor section 114, and an output terminal 115. More specifically, the electrode section 112 includes massage head arms 403R, 403L functioning as electrodes, and a transformer 112c.

The pair of massage head arms 403R, 403L are disposed so as to be approached or touched by the user, and are also provided to receive high frequency signals from the oscillation section 111, and are coupled to the treatment section 40.

Outline of operation of sensor section 100 is explained. The oscillation section 111 supplies high frequency signals, and the massage head arms 403R, 403L receive high frequency signals by way of transformer 112c for isolation and real number conversion. More specifically, both ends of primary coil of the transformer 112c are connected to reflected wave sensor section 113 and grounding potential, and both ends of secondary coil are connected to the massage head arms 403R, 403L.

Impedance in massage head arms 403R, 403L varies with the motion or shape of the user as described below, and the high frequency signal energy consumed in the electrodes varies also depending on the motion and shape of the user. Accordingly, the fluctuation of high frequency signal energy not consumed in the electrodes is sensed by the reflected wave sensor section 113 as fluctuation of reflected wave level. The sensed fluctuation of reflected wave level is amplified and processed in the signal processor section 114, and output from the output terminal 115 as bio-signal. The output bio-signal is given to the CPU 13a (refer to Fig. 7) for controlling the massager 1.

As shown in Fig. 2, the massage head arms 403R, 403L are disposed at the reverse side of surface member of the back rest portion 10a of the massager 1, and other circuit elements, that is, the transformer 112c, oscillation section 111, reflected wave sensor section 113, and signal processor section 114 shown in Fig. 1 are integrally assembled in the sensor section 100, which is disposed in the treatment section 40 inside the back rest portion 10a (refer to Fig. 6). Thebio-signaloutput from the output terminal 115 of the sensor section 100 is, although not shown, given to the CPU 13a (refer to Fig. 7) of the control section 13 disposed inside the massager 1.

Next, the configuration of oscillation section 111 of the sensor section 100 shown in Fig. 1 is explained.

The oscillation section 111 has an oscillator 111a such as crystal oscillator, and an oscillating circuit for outputting high frequency pulse signals is composed. It further includes two low pass filters (not shown) for converting the pulse signals into sinusoidal waves, and attenuators (not shown).

Only when sinusoidal wave signal is output as high frequency output of oscillation section 111, total energy of sinusoidal wave component is consumed in the electrodes when impedance is matched between the oscillation section 111 and the massage head arms 403R, 403L, and hence the reflected wave level from the electrodes becomes zero. In other words, unless sinusoidal wave signal is output, whether impedance is matched or not, energy of non-sinusoidal wave component is always reflected from the electrodes working as the load.

High frequency component may be properly selected in frequency, preferably in a range of 10 MHz to 300 MHz. In this embodiment, it is noted that frequency is set at 40.68 MHz, but it is not limited, and, for example, 13.56 MHz or 27.12 MHz is also a preferred frequency.

Next, the configuration of reflected wave sensor section 113 of the sensor section 100 shown in Fig. 1 is explained.

The reflected wave sensor section 113 includes a terminal $P_1$ for coupling with oscillation output of oscillation section 111, a terminal $P_2$ for coupling with input end (one end of primary coil of transformer 112c) of the electrode section 112, and M coupling circuits 113a, 113b of known structure.

The M coupling circuit 113a is composed of primary coil N1a inserted between the terminals $P_1$ and $P_2$ and secondary coil N2a, and a resistor R connected parallel to both ends of the secondary coil N2a. The M coupling circuit 113b is composed of primary coil N1b inserted between terminal $P_2$ and grounding potential and secondary coil N2b. It is noted that the secondary coils N2a, N2b are connected in series between the grounding potential and the output end of the reflected wave sensor section 113.

Principle of detection of the bio-signal by the reflected wave sensor section 113 is specifically described below.

For example, in the chair type massager 1 shown in Fig. 2, suppose that the user sits correctly on the back rest portion 10a, that is, the shoulder positions of the user are located near the massage head arms 403R, 403L.

When the high frequency sinusoidal wave signal supplied from the oscillation section 111 is supplied to these electrodes, a high frequency current flows between the two massage head arms 403R, 403L, by way of the surface member of the massager 1, clothes of the user, and body tissues of the user (mainly fat components).

When the user approaches or touches the massage head arms 403R, 403L, the reflected wave sensor section 113 senses impedance changes occurring in the massage head arms 403R, 403L as the electrodes receiving high frequency signals.

Change of distance between electrode surface and body surface due to body motion, or difference in distance from electrode surface and body tissue due to body type of the user corresponds to change in imaginary number component of high frequency impedance in the electrodes, while deformation of body tissue by body motion corresponds to change in real number component of high frequency impedance.

Depending on the back side shape of the seated user, the former distance change or latter tissue change occurs periodically, and change of high frequency impedance depending on the body motion or body type of the user occurs in the electrodes.

In other words, the reflected wave sensor section 113 generates and supplies a signal showing change of reflected wave level of high frequency signal corresponding to impedance change occurring between the massage head arms 403R and 403L depending on the body type of the user.

The reflected wave sensor section 113 may be also designed to output a reflected wave signal corresponding to the impedance change of real number and imaginary number occurring between the massage head arms 403R and 403L depending on the body type of the user.

Generally, impedance Z is expressed as follows.

$$Z = R + j(\omega L - (1/\omega C)) \qquad (equation\ 1)$$

R: resistance, L: inductance

C: capacity, $\omega$: angular vibration frequency

The high frequency signal supplied from the oscillation section 111 is totally consumed in the massage head arms 403R, 403L when the impedance is matched between the massage head arms 403R and 403L, but depending on impedance change in such electrodes, the energy consumed in the electrodes varies, and an extra energy returns as reflected wave to the oscillation section 111 from the electrode section 112 through the reflected wave sensor section 113. Herein, the M coupling circuits 113a, 113b of the reflected wave sensor section 113 constitute a directional coupler, and part of energy not consumed in the electrodes is taken out, and given to the signal processor section 114 in a later stage as reflected wave output.

The M coupling circuit using coil is a mere example of known directional coupler, and the reflected wave sensor section 113 maybe also composed by using capacitor, microstrip line, and others.

Next, the configuration of the signal processor section 114 of the sensor section 100 shown in Fig. 1 is described below.

The signal processor section 114 includes an amplifier 114a and a filter 114b. As mentioned above, the signal processor section 114 receives a signal showing change of reflected wave level in the massage head arms 403R, 403L from the reflected wave sensor section 113, and the signal is amplified, and passed through the filter of a proper preset constant, so that a signal corresponding to the body type of the user is taken out.

In other words, the signal processor section 114 processes the signal corresponding to the sensed impedance change, into a desired signal corresponding to the bio-signal.

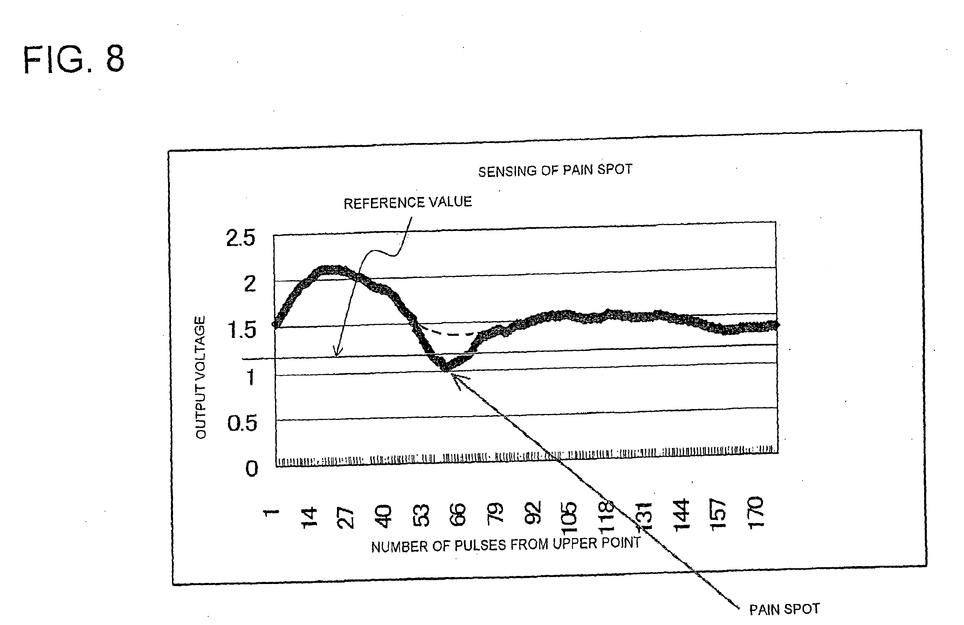

The treatment section 40 having the massage head arms 403R, 403L descends from the upper part to the lower part of the back rest portion 10a depending on the number of pulses of the motor, by the elevating treatment unit 20. In this embodiment, by sensing the signal in every number of motor ascending and descending pulses, a fluctuation graph of output voltage of the signal as shown in Fig. 8 is obtained.

The sensed signal is sent to the control section 13 of the massager 1 sequentially, and the position of discomfort site (pain spot) is judged in the CPU 13a, and the massaging operation is controlled according to this information.

In the massager according to the above-described embodiment, the sensing of the position of discomfort site, shoulder positions and other measurement, and control of massage operation are explained by referring to the following examples.

[Example 1]

(Sensing method of discomfort site (pain spot))

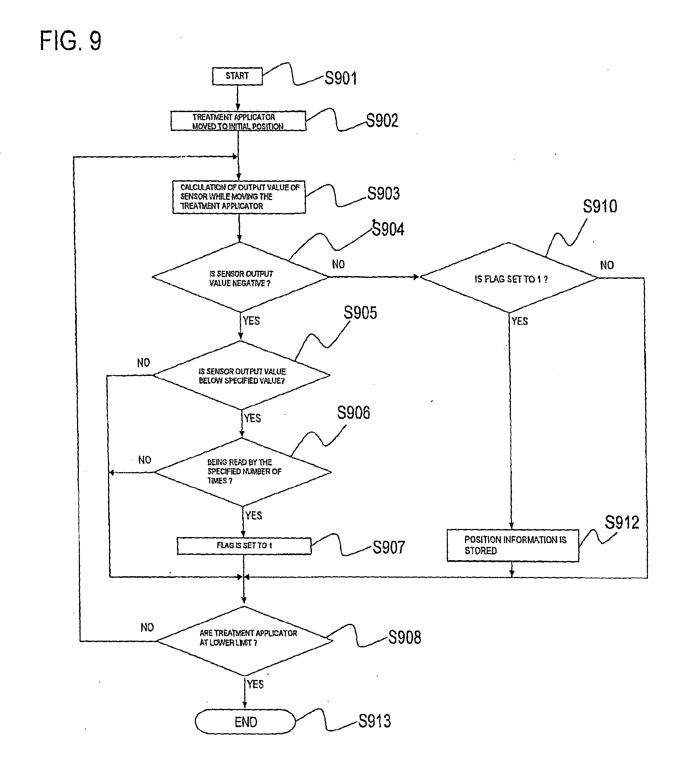

Fig. 9 is a flowchart of operation of massager when sensing the discomfort site (pain spot) of the user.
When the massager 1 according to the embodiment is not engaged in massaging operation, the treatment section 40 is placed in a standby state in an upper portion of the back rest portion 10a so as not to contact with the back of the user when the user sits on the massager 1.
When the user turns on the power source of the massager 1 and presses the start button of massaging operation, the operation of the massager 1 begins (step S901).
Next, the treatment section 40 having the massage heads (treatment applicators) 402a to 402d moves to initial position (step S902).
Then, by moving the treatment section 40, the massage head arms 403R, 403L pivotally supporting the massage heads 402a to 402d move gradually downward from above. The sensor section 100 senses the change of impedance occurring in the massage head arms 403R, 403L functioning as electrodes, as change in output voltage (step S903).
The treatment section 40 is away from the back of the user in initial position, and the output voltage corresponding to the impedance occurring in the massage head arms 403R, 403L is nearly zero. As the treatment section 40 descends on the back side of the user, the output voltage changes according to the body type of the user. Usually, the impedance occurring in the massage head arms 403R, 403L is smaller as the massage head arms 403R, 403L are pressed more firmly to the back of the user, in other words, as the back profile of the user is protruding more to the back rest portion side, and more current flows, and hence the output voltage is larger according to this value.
Hence, the output voltage changes depending on the body type and body fat percentage of the user, and the output voltage increases monotonously in initial phase of measurement (refer to Fig. 8).
When the treatment section 40 passes the shoulder position of the user, the output voltage reaches the peak, and then decreases, and the value of output voltage changes up and down within the measuring range according to the back shape of massaged area of the user and distribution of fat (muscles) of the user.
In this process, if the user feels that the pressure to the discomfort site by the massage heads 402 is too strong (for example, painful position by the individual user, or painful position due to wound or bruise), the user tries to move away from the back rest portion 10a. In such a case, the output voltage from the sensor section 100 may become smaller (refer to solid line in Fig. 8) than the ordinary output voltage (refer to dotted line in Fig. 8).
Alternatively, in the shoulder blade or other site particularly protruding toward the back rest portion 10a, it may be felt painful by massage by usual pressure. The shoulder blade is mostly made of bone with less fat and muscle, the current hardly flows in the bone, and if the user does not move away from the back rest portion 10a, the output voltage from the sensor section 100 is smaller than the ordinary output voltage (refer to dotted line in Fig. 8).
In such a case, the massager 1 of this example judges the position of discomfort site from fluctuation of output voltage from the sensor section 100. In the subsequent operation, such discomfort site is not massaged. Or the operation of the massager is controlled to lower the pressure on such site.
In this example, output value V21 of the sensor section 100 is compared with previous output value V20 of the sensor section 100 stored in the ROM 13b or RAM 13c (step S904). In the case of a first output value, there is no previous value, and it may be compared with an initial value (for example, 0) preset in the storage means.
It is then judged whether the treatment section 40 has passed the shoulder position or not. When output value V21 of the sensor section 100 is smaller than previous output value V20 (YES), it is judged that the treatment section 40 has passed the shoulder position. On the other hand, if output value V21 of the sensor section 100 is larger than previous output value V20 (NO), the treatment section 40 has not passed the shoulder position, and discomfort site is not judged.
When the CPU 13a judges that output value V21 is smaller (-) than output value V20 (YES), output value V21 as signal according to bio-signal from the sensor section 100 is compared with predetermined reference value V22 stored in the ROM 13b or the like (step 5905).
To compare with reference value V22, the reason is as follows. When the treatment section 40 passes the first peak point of shoulder position, the output voltage of the sensor section 100 once decreases, and then changes up and down within the measuring range depending on the back shape of the massaged area of the user and distribution of fat (muscle) of the user. Accordingly, if the change of output value of the sensor section 100 becomes -, it is hard to judge whether it is due to body type of the user or distribution of fat (muscle), or due to reaction of the user moving away from the back rest portion 10a by pressure on discomfort site by the massage heads 402.
Hence, if the output value of the sensor section 100 is not lower than the predetermined reference value (NO), the position of discomfort site of the user is not judged. That is, if the change of output value of the sensor section 100 becomes - within the measuring range due to the body type of the user, it avoids wrong judgment of discomfort site, and the massaging machine is prevented from massaging on the basis of wrong information of discomfort site.

On the other hand, if the output value V21 of the sensor section 100 is lower than the predetermined reference value V22 (YES), it is judged that the user has moved away from the back rest portion 10a due to pressure on discomfort site by the massage heads 402.

At step S906, it is judged if output value V21 has been read as many times as the specified number of times N. Accordingly, if output value V21 of the sensor section 100 satisfies the value at step S904 or S905 by mistake before reaching the discomfort site of the user, it is prevented from judging the position of discomfort site before completion of measurement in the preset range depending on the ordinary human body type.

The specified number of times of reading N in this example is proportional to the number of pulses when driving the motor to move the treatment section 40 up and down. Therefore, if the number of times of measurement calculated from the number of pulses of driving the motor is smaller than the specified number of times of reading N, the process returns to step S903.

On the other hand, when the number of times of measurement calculated from the number of pulses of driving the motor has reached the specified number of times of reading N, flag is set to 1 at step S907 so as to judge the position of discomfort site.

It is then judged if the position of massage heads 402 has reached the lower limit of measuring range or movable range in massaging operation (step S908). If the massage heads 402 do not reach the lower limit, the process returns to step S903 again, and the massage heads 402 are moved by predetermined distance, and output value V21 of the sensor section 100 is measured.

At step S904, then output value V21 is compared with previous output value V20. When the CPU 13a judges that output value V21 is smaller than output value V20, steps S905 to S908 are repeated. On the other hand, when the CPU 13a judges that output value V21 is larger than output value V20 (NO) , it is judged if flag is set to 1 or not (step S910) .

If flag is not set to 1 (NO) , it means that at least one of the conditions at steps S904 to S906 is not satisfied, and the process returns to step S908 or S903, and the massage head arms 403R, 403L are moved by predetermined distance, and the output value of the sensor section 100 is measured again at this position.

On the other hand, when flag is set to 1 (YES), it means that all conditions at steps S904 to S906 are satisfied, by detecting the position of the output value of the sensor once decreased to be lower than the predetermined reference value and then increased, that is, the extreme value of signal depending on the bio-signal in the predetermined range by moving the treatment section 40, the position of discomfort site of the user can be judged. More specifically, in this example, the extreme value is the minimum value (downward hump position) sensed after the first peak value sensing the shoulder position, and this position is judged to be discomfort site, and the position information is written into the storage means (step S912).

At step S908, it is judged if the massage heads 402 have reached the lower limit or not, and if not reaching yet, going back to step S903, position of next discomfort site is sensed. If reaching already, sensing of discomfort site is over (step S913). Therefore, the massager 1 of the example can judge a plurality of discomfort sites.

Thus, by measuring while moving the massage head arms 403R, 403L functioning as electrodes by a predetermined distance on the back side of the user seated against the back rest portion 10a, output voltages output from the sensor section are detected, and a characteristic curve having a peak at the number of ascending and descending pulses corresponding to the shoulder position or discomfort site is obtained as shown in Fig. 8. According to the example, the position of discomfort site can be judged from the number of ascending and descending pulses until reaching the peak corresponding to discomfort site.

Therefore, according to the massager of the example, since the position of discomfort site is judged automatically without setting by the user, the user needs no extra effort, and can enjoy a comfortable massaging operation, while evading the discomfort site for the user or weakening the pressure on the discomfort site.

Besides, since the massage head arms are also used as electrodes, no extra parts are needed for sensing, and a massager of low cost is presented in a simple structure.

Moreover, since the position is sensed according to the number of ascending and descending pulses of driving the motor for moving the massage head arms up and down, the discomfort site can be judged at high precision.

[Example 2]

(Correction method of position of discomfort site 1)

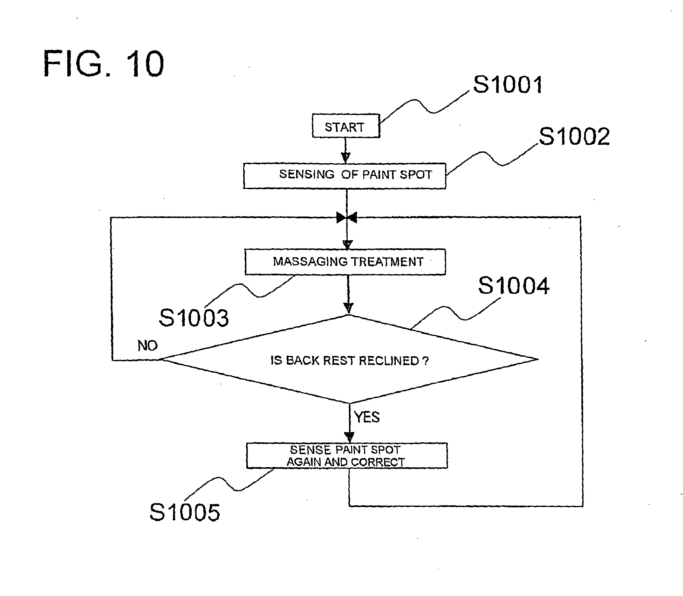

Fig. 10 is a flowchart of correction operation of position of discomfort site of the user in the massager.

The massager 1 according to this embodiment starts massaging operation (step S1001) , and judges the position of discomfort site according to the method, for example, explained in example 1 (step S1002).

Next, the massager 1 massages the user according to the judged position of discomfort site (step S1003) . During this massage treatment, the user may change the angle of the back rest portion 10a (reclining) to seek a better position for massaging. In this case, the position of discomfort site of the user with respect to the back rest portion 10a is changed, and appropriate massaging operation cannot be executed if continued on the basis of the initially judged position of discomfort site.

In this example, at step S1004, it is judged if the back rest portion is reclined or not. If not reclined (NO), correction of position of discomfort site is not needed, and the massage operation continues in the same setting of discomfort site.

If reclined (YES) , at step S1005, the position of discomfort site is set anew. Specifically, the position of discomfort site is sensed again same as in example 1, and the position of discomfort site can be judged. Alternatively, at the start of the massage operation the shoulder position is sensed and stored in the storage means, and then after the back rest portion is reclined the shoulder position is sensed again, and the position of discomfort site can be corrected on the basis of the newly sensed shoulder position. On the basis of the new judged position of discomfort site, the control section 13 controls the subsequent massaging operation by the treatment applicators.

Otherwise, by the moving angle (inclination) of the back rest portion 10a by reclining action, change of position of discomfort site can be calculated by a operational expression preset in the storage means, and a new position may be judged as discomfort site. On the basis of the corrected position of discomfort site, the control section 13 controls the subsequent massaging operation by the treatment applicators. According to this technique, it is not necessary to move the treatment section 40 to the initial position in order to sense the discomfort site again, and the subsequent.massage treatment time can be shortened.

[Example 3]

(Correction method of position of discomfort site 2)

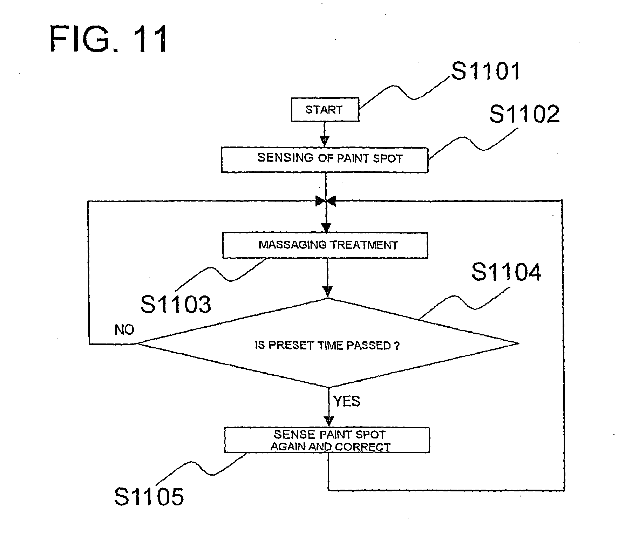

Fig. 11 is a flowchart of correction operation of position of discomfort site of the user in the massager.

The massager 1 of this embodiment starts massaging operation (step S1101), and judges the position of discomfort site according to the method, for example, explained in example 1 (step S1102).

Next, the massager 1 massages the user according to the judged position of discomfort site (step S1103) . In the course of this massage treatment, the user may change the position or move on the massager 1 to seek a better position for massaging. In this case, the position of discomfort site of the user with respect to the back rest portion 10a is changed, and appropriate massaging operation cannot be executed if continued on the basis of the initially judged position of discomfort site.

In this example, by sensing again the position of discomfort site or correcting the shoulder position at every preset time interval, the position of discomfort site is always judged correctly, and appropriate massaging operation is realized. Therefore, at step S1104, it is judged if the preset time has passed or not, and if not passed (NO), correction of position of discomfort site is not necessary, and the massage treatment continues in the same setting of position of discomfort site.

On the other hand, if already passing the predetermined time (YES), at step S1105, the position of discomfort site is set again and corrected. Specifically, the position of discomfort site is sensed again same as in example 1, and the position of discomfort site can be determined. On the basis of the new position of discomfort site, the control section 13 controls the subsequent massaging operation by the treatment applicators. According to this technique, the position of discomfort site is always judged correctly, and appropriate massaging operation is realized.

The invention is described herein by referring to preferred embodiments and examples, but the invention is not limited to the illustrated embodiments and examples alone, but may be modified or combined in various manners.

**Claims**

1. A massager comprising:

    a back rest portion for supporting the back of the user,
    treatment applicators provided in the back rest portion for massaging the user,
    a treatment section for moving a pair of electrodes supporting the treatment applicators according to the massaging operation,
    a bio-signal sensor for sensing bio-signals of the user depending on impedance changes between the pair of electrodes, and
    a control section for controlling the massaging operation of the treatment applicators on the basis of the bio-signals,
    wherein the bio-signal sensor has:

an oscillation section for supplying a high frequency signal,

the pair of electrodes disposed to be approached or touched by the user for receiving the high frequency signal from the oscillation section,

a sensor section for sensing changes of the impedance occurring in the electrodes receiving the high frequency signal when the user approaches or touches the electrodes, and

a signal processor section for processing from a signal corresponding to the sensed impedance changes to a signal depending on a desired bio-signal, and

the control section moves the treatment section and detect an extreme value of signal corresponding to the bio-signal in a predetermined range, and judges the position of discomfort site of the user.

2. A massager according to claim 1, wherein the extreme value is a minimum value sensed after a first peak.

3. A massager according to claim 1 or 2, wherein the control section controls so as not to massage the position of discomfort site when the position of discomfort site is judged.

4. A massager according to claim 1 or 2, wherein the control section controls to weaken the pressure of treatment applicators in massaging operation at the position of discomfort site when the position of discomfort site is judged.

5. A massager according to any one of claims 1 to 4, wherein the control section can judge a plurality of the positions of discomfort site.

6. A massager according to any one of claims 1 to 5, wherein the control section does not judge the position of discomfort site of the user when the extreme value is not lower than a predetermined reference value.

7. A massager according to any one of claims 1 to 6, wherein the control section judges the position of discomfort site again when the inclination of the back rest portion is changed, and controls the massaging operation by the treatment applicator on the basis of the newly judged position of discomfort site.

8. A massager according to any one of claims 1 to 6, wherein the control section corrects the position of discomfort site, when the inclination of the back rest portion is changed, according to the changed inclination, and controls the massaging operation by the treatment applicator on the basis of the corrected position of discomfort site.

9. A massager according to any one of claims 1 to 6, wherein the control section judges the position of discomfort site again in a predetermined time after judging the position of discomfort site, and controls the massaging operation by the treatment applicator on the basis of the newly judged position of discomfort site.

10. A massager according to any one of claims 1 to 9, wherein the electrodes are arms rotatable with respect to the treatment section for supporting the plurality of treatment, applicators, and

thepluralityof treatment applicators are disposed in a line nearly parallel to the moving direction of the treatment section on both sides of the center of rotation of the arms.

11. A massager according to any one of claims 1 to 10, wherein the sensor section generates and supplies a signal expressing changes of reflected wave level of the high frequency signal corresponding to the impedance changes occurring between the electrodes depending on the body type of the user.

12. A massager according to any one of claims 1 to 10, wherein the sensor section outputs a reflected wave signal depending on impedance changes of real number and imaginary number occurring between the electrodes depending on the body type of the user.

13. A massager according to any one of claims 1 to 12, wherein the frequency of the high frequency signal is in a range of 10 MHz to 300 MHz.

# FIG. 1

EP 1 629 818 A1

## FIG. 2

FIG. 3

FIG. 4

FIG. 5

FIG. 6

FIG. 7

FIG. 8

SENSING OF PAIN SPOT

REFERENCE VALUE

PAIN SPOT

OUTPUT VOLTAGE

2.5  2  1.5  1  0.5  0

NUMBER OF PULSES FROM UPPER POINT

1  14  27  40  53  66  79  92  105  118  131  144  157  170

## FIG. 9

```
                    START              S901
                       │
                       ▼
           TREATMENT APPLICATOR         S902
           MOVED TO INITIAL POSITION
                       │
        ┌──────────────┤
        │              ▼
        │   CALCULATION OF OUTPUT VALUE OF    S903
        │   SENSOR WHILE MOVING THE
        │   TREATMENT APPLICATOR
        │              │                  S904
        │              ▼
        │          ◇ IS SENSOR OUTPUT ◇────NO────► ◇ IS FLAG SET TO 1 ? ◇────NO───┐   S910
        │            VALUE NEGATIVE ?                                              │
        │              │                                   │YES                    │
        │             YES              S905                 │                       │
        │              ▼                                    │                       │
        │  NO  ◇ IS SENSOR OUTPUT VALUE ◇                   │                       │
        │ ◄──── BELOW SPECIFIED VALUE?                      │                       │
        │              │                S906                │                       │
        │             YES                                   ▼          S912         │
        │              ▼                        POSITION INFORMATION IS             │
        │  NO  ◇ BEING READ BY THE ◇              STORED                            │
        │ ◄──── SPECIFIED NUMBER OF                         │                       │
        │        TIMES ?                                    │                       │
        │              │                                    │                       │
        │             YES                                   │                       │
        │              ▼                                    │                       │
        │      FLAG IS SET TO 1     S907                    │                       │
        │              │                                    │                       │
        │              ├◄───────────────────────────────────                       │
        │              │◄─────────────────────────────────────────────────────────┘
        │              ▼
        │ NO  ◇ ARE TREATMENT APPLICATOR ◇   S908
        └──── AT LOWER LIMIT ?
                       │
                      YES
                       ▼
                  (  END  )          S913
```

# FIG. 10

S1001

START

S1002

SENSING OF PAINT SPOT

MASSAGING TREATMENT

S1003

S1004

NO ◇ IS BACK REST RECLINED ?

YES

SENSE PAINT SPOT
AGAIN AND CORRECT

S1005

# FIG. 11

```
                                    S1101
                      ┌─────────┐  ╱
                      │  START  │◁╱         S1102
                      └─────────┘          ╱
                           ▼              ╱
              ┌──────────────────────────┐
              │   SENSING OF PAINT SPOT   │◁
              └──────────────────────────┘
                           ▼
      ┌────────────────────────────────────────────┐
      │        ┌──────────────────────────┐        │
      │        │    MASSAGING TREATMENT    │        │
      │        └──────────────────────────┘        │
      │                    ▼              S1104     │
      │  S1103                           ╱          │
      │      NO        ╱◇◇◇◇◇◇◇◇◇◇◇◇◇╲   ╱           │
      │◁─────────────◇ IS PRESET TIME PASSED ? ◇────┤
      │                ╲◇◇◇◇◇◇◇◇◇◇◇◇◇╱              │
      │                       ▼                     │
      │                      YES                    │
      │        ┌──────────────────────────┐        │
      │        │    SENSE PAINT SPOT       │        │
      │        │    AGAIN AND CORRECT      │────────┘
      │        └──────────────────────────┘
      │     ╱
      └── S1105
```

22

**EP 1 629 818 A1**

| | | | |
|---|---|---|---|
| **European Patent Office** | **EUROPEAN SEARCH REPORT** | **Application Number** EP 05 01 8709 | |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 01/37729 A (OMRON CORPORATION; KASAI, EIJI; FUKUI, RYO) 31 May 2001 (2001-05-31) * figures 1-6 * ----- | 1-13 | A61H1/00 A61H37/00 A61B5/053 |
| A | PATENT ABSTRACTS OF JAPAN vol. 2000, no. 15, 6 April 2001 (2001-04-06) -& JP 2000 342644 A (YA MAN LTD), 12 December 2000 (2000-12-12) * abstract; figures * ----- | 1-13 | |
| A | EP 1 230 904 A (SANYO ELECTRIC CO., LTD) 14 August 2002 (2002-08-14) * the whole document * ----- | 1-13 | |
| A | US 2002/123704 A1 (HORI KUNIHIKO ET AL) 5 September 2002 (2002-09-05) * the whole document * ----- | 1-13 | |
| A | EP 0 989 671 A (OMRON HEALTHCARE CO., LTD) 29 March 2000 (2000-03-29) * the whole document * ----- | 1-13 | TECHNICAL FIELDS SEARCHED (IPC) A61H A61B |

The present search report has been drawn up for all claims

| Place of search | Date of completion of the search | Examiner |
|---|---|---|
| Munich | 23 November 2005 | Elmar Fischer |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................

& : member of the same patent family, corresponding document

EPO FORM 1503 03.82 (P04C01)

23

**ANNEX TO THE EUROPEAN SEARCH REPORT
ON EUROPEAN PATENT APPLICATION NO.**                    EP 05 01 8709

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2005

| Patent document cited in search report | | Publication date | Patent family member(s) | | Publication date |
|---|---|---|---|---|---|
| WO 0137729 | A | 31-05-2001 | AU<br>CN<br>JP | 1416301 A<br>1391450 A<br>3575463 B2 | 04-06-2001<br>15-01-2003<br>13-10-2004 |
| JP 2000342644 | A | 12-12-2000 | NONE | | |
| EP 1230904 | A | 14-08-2002 | CN<br>JP<br>TW<br>US | 1368040 A<br>2002233558 A<br>508239 B<br>2002138023 A1 | 11-09-2002<br>20-08-2002<br>01-11-2002<br>26-09-2002 |
| US 2002123704 | A1 | 05-09-2002 | TW | 510789 B | 21-11-2002 |
| EP 0989671 | A | 29-03-2000 | JP<br>JP<br>US | 3484355 B2<br>2000098048 A<br>6545614 B1 | 06-01-2004<br>07-04-2000<br>08-04-2003 |

EPO FORM P0459